# EUROPÄISCHE PATENTANMELDUNG

(11) **EP 1 820 441 A1**
(43) Veröffentlichungstag der Anmeldung: **22.08.2007**
(21) Anmeldenummer: 06003127.5
(22) Anmeldetag: 16.02.2006
(51) Int. Cl.: A61B 5/00

(54) **Mikronadelanordnung mit Sensor basierend auf der abgeschwächten Totalreflexion (ATR)**

(71) Anmelder: Roche Diagnostics GmbH, 68305 Mannheim (DE); F.HOFFMANN-LA ROCHE AG, 4070 Basel (CH)
(72) Erfinder: Mischler, Reinhold, 67063 Ludwigshafen (DE); Werner, Gerhard, 69469 Weinheim (DE)
(74) Vertreter: Rauscher, Andreas

(57) **Zusammenfassung**

Es wird ein Sensor zur optischen Bestimmung der Konzentration eines Analyten in einer Körperflüssigkeit beschrieben. Dieser Sensor beinhaltet eine Nadelanordnung, die mindestens eine Hohlnadel, mit einem sich von dem distalen Ende zum proximalen Ende erstreckenden Hohlraum, aufweist, deren distales Ende zum Stechen geeignet ist. Das proximale Ende der Nadel mündet in eine Kammer, in der Flüssigkeit gesammelt werden kann, die durch das distale Nadelende eintritt. Ein Fenster das für Infrarotstrahlung zumindest zum Teil durchlässig ist, tritt in direkten Kontakt mit der Kammer.

## Beschreibung

### Technisches Gebiet der Erfindung

Die Erfindung liegt im Gebiet der Diagnostik und im speziellen handelt es sich um schmerzarme Gewinnung von Flüssigkeit zur optischen Messung von Blutbestandteilen.

Zur Detektion von Analyten wie Glukose gibt es vielfältige Analysesysteme. Hierbei wird dem Patienten Blut entnommen und auf einen Testträger gebracht, um dann in einem Messgerät separat von der Blutentnahme vermessen zu werden. Hierzu dienen Messgeräte sowohl mit optischer Detektion als auch mit elektrochemischem Nachweis. Diese Systeme weisen in den meisten Fällen einen Teststreifen auf, auf dem der Patient einen Bluttropfen deponieren muss.

In dem US Patent mit der Nummer US 6,603,987 ist eine Kombination beschrieben, bei der ein Nadelarray verwendet wird, das mit einem Teststreifen verbunden ist. Der Nachteil dieser Methode ist, dass sie nicht zur kontinuierlichen Messung verwendet werden kann, da der Teststreifen nur für eine Messung benutzt werden kann. Das bedeutet für den Patienten, dass er sich mehrmals am Tag mit dem Nadelarray stechen muss.

Die US Anmeldung mit der Nummer US 2003/0185384 beschreibt die Produktion von Mikronadeln, sowie die Verknüpfung eines Nadelarrays mit einem Sensorelement. In diesem Fall handelt es sich um ein elektrochemisches Nachweiselement, das über ein Transfermedium (beispielsweise einem hydrophilen porösen Material, das die Flüssigkeit aufnimmt) mit dem Nadelarray verbunden ist. Auch dieses System weist den Nachteil auf, dass es nicht zur kontinuierlichen Überwachung des Patienten dienlich ist. Daraus ergibt sich für den Patienten wiederum der Nachteil, dass er sich mehrfach täglich stechen muss.

Aufgrund der verwendeten Reagenzien für die Analyse, wie sie im Stand der Technik beschrieben ist, ist das Testelement nur für eine Messung zu verwenden, da sich die Reagenzien bei der Reaktion mit dem Analyten verbrauchen. Dies bedeutet für den Patienten, dass er das Testelement nach jeder Messung austauschen muss. Neben den Arbeitsschritten, die mit dem Austausch verbunden sind, ist ein weiterer Nachteil, dass das System nur begrenzt miniaturisiert werden kann, wenn das System für den Patienten handhabbar bleiben soll.

Aus diesen Nachteilen des Standes der Technik stellt sich die Aufgabe, ein miniaturisiertes Analyesystem vorzuschlagen, das kontinuierlich eine Analyse von Körperflüssigkeit des Patienten vornehmen kann.

Im Rahmen der Erfindung wird eine Nadelanordnung vorgeschlagen, die mindestens eine Nadel aufweist, deren distales Ende zum Stechen geeignet ist und deren proximales Ende in eine Kammer mündet. In einer bevorzugten Ausfiihrungsform wird ein Array von Nadeln benutzt. Dieses Nadelarray kann aus 2 bis mehreren hundert Nadeln bestehen. Die Anordnung der Nadeln kann in einer Reihe oder in einer zwei dimensionalen Matrix vorgenommen werden und die Anordnung der Nadeln kann dabei randominisiert sein. Dieses Array kann eckig oder auch rund ausgeführt sein.

Eine bevorzugte Form der Nadelanordnung ist eine längliche, rechteckige Anordnung. Diese Nadelanordnung wird kombiniert mit einem Fenster, das für Infrarotstrahlung zumindest zum Teil durchlässig ist. Die Kammer bildet dabei einen Zwischenraum zwischen Nadelanordnung und Fenster. Eine durch das Fenster geleitete Strahlung kann so mit der Flüssigkeit in der Kammer wechselwirken. Die Nadeln der Nadelanordnung sind vorzugsweise hohl und münden mit ihrem proximalen Ende des Hohlraums der Nadeln in die Kammer. Den proximalen Enden der Nadeln gegenüber ist das Fenster angeordnet. Das Fenster ist mindestens in einem Teil des Wellenlängenbereichs von 500 - 20.000 nm lichtdurchlässig. Ein bevorzugter Wellenlängenbereich ist von 2.000 - 12.000 nm. Die Schichtdicke des Fensters kann bevorzugter weise 0,5 mm betragen. In einer besonders bevorzugten Ausführungsform kann die Schichtdicke 0,025 - 0,2 mm betragen. Das Volumen der Kammer kann zwischen 10 und 1.000 nl betragen, je nach Anzahl der Nadeln und Geometrie der Kammer. Die Nadeln sind bevorzugter weise durch Stege miteinander verbunden. Die Nadelspitzen sind so geformt, dass sie nur so weit in die Haut eindringen, dass bevorzugt interstitielle Flüssigkeit gewonnen wird. Das bedeutet, dass die Nadelspitzen nur wenige 100 µm bis zu 1 mm lang sind. Die Nadeln besitzen einen Hohlraum durch den die Körperflüssigkeit in die Kammer eindringt. Zwischen dem Austritt des Nadelhohlraums in die Kammer und dem Kammerraum kann sich eine Filtermembran befinden. Diese Membran verhindert das Eindringen von größeren Proteinen in die Kammer. Diese Membran besteht bevorzugter Weise aus Polymeren oder Silizium.

Auf der gegenüberliegenden Seite der Membran befindet sich die Kammer die durch das Fenster abgeschlossen wird. Dieses Fenster besteht bevorzugter Weise aus Infrarot durchlässigen Materialien wie z. B. Silber, Silberhalogeniden, Zinkselenid, Diamant, Germanium oder Silizium. Falls nicht Diamant als Hauptmaterial verwendet wird, kann eine Beschichtung des Fenstermaterials mit Diamant vorgenommen werden. Dies verhindert eine Anlagerung von Proteinen an der Fensteroberfläche. Diese Anlagerung von Proteinen kann zu einer Abschwächung des Signals führen. Das Fenster kann verschiedene Geometrien einnehmen. Dabei kann das Nadelarray eine andere Geometrie und Dimension aufweisen als das Fenster. So ist eine Kombination eines runden Nadelarrays mit einem eckigen Fenster möglich. Eine bevorzugte Ausführungsform ist ein rechteckiges Fenster, das sich über die, in ein beziehungsweise zwei oder mehr Reihen angeordneten Nadeln, erstreckt. Eine bevorzugte Größe dieses Arrays beträgt 0,01 bis 25 mm², eine besonders bevorzugte Ausführungsform liegt im Größenbereich von 0,01 bis 0,25 mm².

An einem Ende des Fensters befindet sich ein Strahler (z. B. ein thermischer Infrarot-strahler wie Dünnfilmstrahler). Auf der gegenüber liegenden Seite des Strahlers kann sich am anderen Ende des Fensters ein Detektor befinden wie z. B. ein pyroelektrisches Detektor Array mit Interferenzfiltern. Die Strahlung die durch den Strahler in das Fenster eingestrahlt wird, ist bevorzugter weise monochromatisch. Mit Hilfe dieser Strahlung wird an den Fensterseiten ein evaneszentes Feld aufgebaut, das mit der Flüssigkeit in der Kammer wechselwirkt. Bei Verwendung von monochromatischer Strahlung findet eine Wechselwirkung mit nur ganz bestimmten Molekülkonfigurationen statt. Dadurch wird gewährleistet, dass nur Moleküle detektiert werden, die bestimmte Molekülgruppen aufweisen, wie beispielsweise Glukosemoleküle. Aufgrund dieser Wechselwirkung wird die Energie des Lichtes abgeschwächt. Diese Energie- beziehungsweise Lichtabsorption wird mit Hilfe des Detektors ermittelt. Zwischen der Menge der absorbierten Strahlung besteht eine direkte Korrelation zur Analytkonzentration. Aufgrund dieser Korrelation kann die Konzentration des Analyten bestimmt werden. Die Verwendung der Lichtabschwächung durch ein evaneszentes Feld zur Analytbestimmung ist unter der der Bezeichnung ATR (Attenuated Total Reflection) bekannt ( A. Bittner et.al.; Mikrochim Acta [Suppl.] 14, 827-828 (1997))und es wird auf diese Referenz Bezug genommen.

In einer bevorzugten Ausführungsform wird das Analysesystem mittels eines Haftmediums auf die Haut des Patienten aufgebracht. Dieses Haftmedium kann beispielsweise ein Pflaster sein, in dem das Analysesystem integriert ist. Durch etwas Druck auf das Pflaster werden die Nadelspitzen des Nadelarrays in die Haut gedrückt und es kann Flüssigkeit aus der Haut in die Kammer fließen. Um Energieversorgung und Datenübertragung in dieses abgeschlossene System zu gewährleisten, können sowohl Strahler wie auch Detektor so ausgewählt werden, dass sie mechanisch von Steuerelementen abgekoppelt sind. Der Datenaustausch findet dann über elektrische Schnittstellen mit einem separaten Steuer- und Messgerät statt. Eine weitere Möglichkeit ist, die Ankopplung von Lichtleitern an das Fenster, wodurch eine geringere Möglichkeit zur Miniaturisierung ermöglicht wird.

Ein Vorteil dieses Sensors ist die lange Lagerfähigkeit sowie die leichte Sterilisierbarkeit. Dies hat seinen Grund in der rein optischen Detektion ohne Verwendung von Chemikalien mit beschränkter Haltbarkeit und eingeschränkter Aktivität nach Sterilisation. In dem beschriebenen Sensor braucht hierauf keine Rücksicht genommen werden und es kann der komplett zusammengebaute Sensor sterilisiert werden. Seine Haltbarkeit ist nicht durch empfindliche Reagenzien begrenzt.

Des Weiteren wird ein System beansprucht, das aus einer Lichtquelle zur Einkopplung der Strahlung in das Fenster, einem Detektor zum Auffangen der Strahlung an der gegenüberliegenden Seite des Fensters und zur Generierung von Messsignalen sowie einem Auswertegerät zur Berechnung der Konzentration des Analyten aus den Messsignalen besteht. Dieses System kann in eine pflasterähnliche Struktur integriert sein, so dass beim Aufbringen des Pflasters auf die Haut, die Nadelspitzen des Nadelarrays in die Haut gedrückt werden.

Das System kann dabei Strahler wie auch Detektoren besitzen, die über elektrische Schnittstellen, wie beispielsweise Infrarot- oder Funk- Schnittstellen, steuerbar und auslesbar sind. In solch einem System ist keine weitere Kontaktierung der elektrischen und optischen Einheiten nötig. Der Patient kann sich frei bewegen und das System ist gegenüber äußeren, mechanischen Einflüssen durch das Pflaster abgeschirmt. Die altemative Ankopplung des Strahlers beziehungsweise Detektors über Lichtleiter bedeutet ein voluminöseres und offeneres System, das mechanischen Einflüssen gegenüber nicht komplett abgeschirmt werden kann.

Vorteile des erfindungsgemäßen Sensors sind der Verzicht auf Reagenzien bei der Analyse der Probe. Hierdurch ist eine hohe Integration der Komponenten möglich, was wiederum eine gute Miniaturisierung des Systems ermöglicht. Für den Patienten ist der Sensor wie auch das System leicht handhabbar, da außer des Anbringens des Sensors auf der Haut nur wenige Handhabungsschritte nötig sind. Der Sensor wie auch das System können einfach sterilisiert werden und lange haltbar gemacht werden, da keine empfindlichen Chemikalien bzw Reagenzien für die Analyse nötig sind. Durch den Verzicht auf Reagenzien ist die Gefahr einer Kontamination des Patienten durch Chemikalien sehr gering.

### Detaillierte Beschreibung

Erfindungsgemäß wird ein Sensor beschrieben, der zur optischen Bestimmung von Konzentrationen eines Analyten in einer Flüssigkeit eingesetzt wird. Im Speziellen besteht diese Flüssigkeit aus einer Körperflüssigkeit wie Blut, Plasma, Serum oder Interstitieller Flüssigkeit. Sie wird erfindungsgemäß über ein Nadelarray, mit mindestens einer Nadel gewonnen, die in die Haut des Patienten eindringt.

### Nadel

Dieser Sensor beinhaltet eine Nadelanordnung, die mindestens eine hohle Nadel aufweist, deren distales Ende zum Stechen geeignet ist und deren proximales Ende in eine Kammer mündet, in die Flüssigkeit eindringen kann. Die Nadelspitzen können dabei nur wenige 100 µm bis zu 1 mm lang sein. Der Durchmesser der Nadel beträgt bevorzugter weise nicht mehr als 100 µm. In einer besonders bevorzugten Ausführungsform beträgt der Durchmesser der Nadeln zwischen 20 und 70 µm. Die Nadeln besitzen einen Hohlraum durch den die Körperflüssigkeit in die Kammer eindringt. Der Innendurchmesser der Nadel liegt dabei zwischen 10 und 60 µm, bevorzugter weise zwischen 15 und 40µm. Für diesen Zweck ist die mindestens eine Nadel innen hohl. Die Flüssigkeit wird beispielsweise aus der Haut eines Patienten gewonnen, indem die mindestens eine Nadel in die Haut gedrückt wird. Das distale Ende der Nadel ist dabei so geformt, dass es einen Spitzenbereich aufweist, der sich zu einem Teil über die Nadellänge erstreckt und in einer bevorzugten Ausführungsform bis zur Einmündung der Nadel in die Kammer ausgedehnt sein kann. Eine alternative Ausführungsform ist eine Nadel die einen Kapillarkanal aufweist, der sich an den Spitzenbereich anschließt und sich über einen Teil der Nadel erstreckt und in einer bevorzugten Ausführungsform über die ganze Nadellänge erstreckt. Dieser Kapillarkanal ist in einem Bereich, der von der Spitze ausgeht und über diese hinausgeht, nach außen offen, so wie er in der Patentanmeldung US 2003 18282 beschrieben ist. Die Nadeln können aus verschiedenen Materialien bestehen, wie z. B. Metall, Silizium, Keramik oder Polymer wie beispielsweise Polycarbonat. Die Nadel ist über ihre Länge von der Spitze bis zur Einmündung in die Kammer hohl.

### Nadelarray

Mehrere solcher hohlen Nadeln können in einem Nadelarray angeordnet sein. Eine bevorzugte Ausführungsform enthält zwischen 10 und 100 Nadeln in einer zweidimensionalen Matrix, es können aber auch mehr oder weniger Nadeln sein. Die Reihen können dabei linear nebeneinander angeordnet sein. Eine bevorzugte Anordnung dieses Arrays ist ein Rechteck beziehungsweise Quadrat. Alternativ können die Nadeln aber auch in einem Kreis oder oval angeordnet sein.

Dabei kann das Nadelarray eine andere Geometrie und Dimension aufweisen als das Fenster. So ist eine Kombination eines runden Nadelarrays mit einem eckigen Fenster möglich, aber auch die umgekehrte Kombination eines eckigen Nadelarrays mit einem runden Fenster. Eine bevorzugte Ausführungsform, die nachfolgend repräsentativ für alle anderen Anordnungen von Fenster und Nadelarray beschrieben wird, ist ein rechteckiges Fenster, das sich über die, in ein beziehungsweise zwei oder mehr Reihen angeordneten Nadeln, erstreckt. Eine bevorzugte Größe dieses Arrays beträgt 0,01 bis 25 mm², eine besonders bevorzugte Ausführungsform liegt im Größenbereich von 0,01 bis 0,25 mm².

### Membran

Zwischen den proximalen Enden der Nadel bzw. des Nadelarrays und der Kammer kann sich eine Membran befinden, die verhindert, dass große Moleküle (>ca. 10 TDa)die Kammer verstopfen oder sich störend auf die Messfläche des Fensters ablagern. Diese Membran besteht bevorzugter Weise aus Polymeren, Cellulose, Silizium oder Polyamid (PA). Eine weitere Möglichkeit stellt eine Kernspurmembran dar, die aus Polycarbonat (PC) besteht. Kernspurmembranen weisen eine hohe mechanische und chemische Stabilität auf. Eine bevorzugte Ausführungsform der Membran weist eine Siliziumschicht auf bzw. besteht aus einer Siliziumschicht, wie sie beispielsweise in der Publikation von Zahn et al. (Microdialysis Microneedles for continous Medical Monitoring; Biom. Microdevices 7:1, 59 - 69, 2005) beschrieben wird. Diese Membran weist sehr geringe Dimensionen auf und kann zudem in einem Prozess zusammen mit den Nadeln und je nach Material alternativ auch mit dem Fenster hergestellt werden.

### Kammer und Volumenstrom

Die Kammer, die sich zwischen dem Nadelarray und dem Fenster erstreckt, dient dazu zumindest einen Teil der gesammelten Körperflüssigkeit mit dem Fenster in Kontakt zu bringen. Die Dimension der Kammer ist dabei so gewählt, dass eine ausreichende Benetzung des Fensters stattfinden kann, aber weiterhin ein guter Austausch zwischen Körperflüssigkeit und Kammerflüssigkeit gewährleistet ist. Dabei kann die Dimension der Kammer sehr unterschiedlich gewählt werden, je nachdem, ob der Flüssigkeitsaustausch zwischen Kammer und Haut ausschließlich über Diffusion stattfindet, oder ob ein künstlicher Flüssigkeitsfluss erzeugt wird. Für den diffusiven Austausch sollte das Volumen der Kammer möglichst klein gewählt werden, um keinen Zeitversatz zwischen gemessener Konzentration in der Kammer und aktueller Konzentration in der Haut zu erzeugen. Ein Volumenbereich in dem der Zeitversatz nur wenige Minuten beträgt liegt zwischen 0,001 und 5 mm³ inklusiv dem Volumen der Nadelhohlräume. Ein bevorzugter Bereich liegt zwischen 0,01 und 0,1 mm³.

Bei einer alternativen Ausführungsform des Sensors, kann das Volumen der Kammer größer gewählt werden, weil ein aktiver Flüssigkeitstransport durch das Nadelarray in die Kammer stattfindet. Dies kann beispielsweise durch die Wahl der Materialien für das Fenster und dessen Dicke ermöglicht werden. Wenn das Fenster aus Silizium gefertigt wird und nicht dicker als 0.025-0.2 mm ist, kann es in seiner Längsausrichtung in Schwingung versetzt werden. Mit dieser schwingenden Bewegung kann die Flüssigkeit in der Kammer bewegt werden. Auf diese Weise kann ein aktiver Flüssigkeitsaustausch zwischen Kammer und Körperflüssigkeit in der Haut des Patienten vorgenommen werden. Die Schwingung des Fensters kann beispielsweise durch ein Piezoelement angeregt und gesteuert werden. Dieses Piezoelement kann auf der Außenseite des Fensters angebracht sein. Alternative Möglichkeiten das Fenster in Schwingung zu versetzen ist eine mechanische Kraftübertragung auf das dünne Fenster. Hierbei wäre die Wahl des Materials nicht auf Silizium beschränkt.

### Optik

Zur Einkopplung von Licht in das Fenster, befindet sich auf mindestens einer Seite des Fensters eine Strahlungsquelle. Bevorzugter weise ist dies eine Seitenfläche des Fensters, die vorwiegend parallel zur Ausrichtung der Nadeln liegt. Besonders bevorzugt ist, bei ungleicher Länge der Seitenflächen eine kurze Seitenfläche zur Lichtentkopplung zu verwenden, um einen möglichst langen Strahlengang oberhalb der Kammer zu haben. Dadurch kann die Menge eingestrahlten Lichtes erhöht werden. Es kann entweder über die gesamte Fläche des Fensters Licht eingestrahlt werden oder es kann durch Anordnung mindestens einer Strahlungsquelle über mindestens einen Teil der Fläche des Fensters Licht eingekoppelt werden.

Diese mindestens eine Strahlungsquelle kann entweder auf das Fenster aufgeklebt werden oder mechanisch über mindestens einen Lichtleiter angekoppelt sein. Wenn die Strahlungsquelle und der Detektor nicht auf das Fenster aufgeklebt sind, kann zur Ankopplung der Strahlungsquelle bzw. des Detektors ein Optomodul dienen. Dabei können der Detektor und/oder die Strahlungsquelle in das Optomodul integriert sein oder über Lichtleiter mit dem Optomodul verbunden sein. In dem Fall in dem die Strahlungsquelle und der Detektor über Lichtleiter mit dem Fenster verbunden sind verbindet das Optomodul die Lichtleiter die zur Lichtquelle bzw. zum Detektor führen mit dem Fenster des Sensors. Das Optomodul gewährleistet dabei eine plane Ankopplung des Lichtleiters an das Fenster. Es besteht aus einer Halterung, die auf der einen Seite mit dem Lichtleiter verbunden ist und auf der anderen Seite mit dem Fenster. Sowohl die Verbindung zum Lichtleiter als auch die zum Fenster ist lösbar. Diese Verbindung kann eine Steck- oder Schraubverbindung sein, oder jede andere wieder lösbare Verbindung. Das Optomodul kann dabei so konfiguriert sein, dass es beide Lichtleiter ankoppeln kann. Auf diese Weise kann die optische Verbindung zwischen dem System und den optischen Modulen, wie Lichtquelle und Detektor gleichzeitig geknüpft und wieder gelöst werden. Das würde den Einsatz für den Patienten erleichtern, der diesen Vorgang mit einer Hand bewerkstelligen könnte.

### Fenster

Das Fenster besteht bevorzugter Weise aus Infrarot durchlässigen Materialien wie z. B. Silber, Silberhalogeniden, Zinkselenid, Diamant, Germanium oder Silizium. Falls nicht Diamant als Hauptmaterial verwendet wird, kann eine Beschichtung des Fenstermaterials mit Diamant vorgenommen werden. Dies verhindert eine Anlagerung von Proteinen an der Fensteroberfläche. Diese Anlagerung von Proteinen kann zu einer Abschwächung des Signals führen. Das Fenster kann verschiedene Geometrien einnehmen.

Zur idealen Einkopplung des Lichtes wird das Fenster an der Ankopplungsseite zu einem idealen Einkopplungswinkel abgeschliffen. Dieser Winkel beträgt zwischen 0° und 60°, bevorzugter Weise zwischen 30° und 50°. Dieser Winkel ist abhängig von der Wellenlänge des eingestrahlten Lichtes, da in dem Fenster Totalreflektion erreicht werden soll. Auf einer anderen, bevorzugter weise dem Strahler gegenüber liegenden Fläche des Fensters, wird das Licht zur Detektion ausgekoppelt. Auch hier gibt es einen idealen Winkel zwischen 0° und 60°, bevorzugterweise zwischen 30° und 50°. Der Detektor kann ebenfalls entweder aufgeklebt oder mechanisch über einen Lichtleiter angekoppelt werden.

### Lichtquelle / Detektor:

Wie bereits erwähnt, kann sowohl monochromatisches Licht als auch polychromatisches Licht eingekoppelt werden. In dem bevorzugten Falle einer monochromatischen Einstrahlung in das Fenster wird beispielsweise ein pyroelektrischer Detektor eingesetzt. Die Einstrahlung von monochromatischem Licht kann entweder durch eine monochromatische Lichtquelle erreicht werden oder durch eine polychromatische Lichtquelle, deren Licht durch Filter auf bestimmte Längenwellen beschränkt in das Fenster eingekoppelt wird. Im Falle der polychromatischen Einstrahlung und Detektion, kann beispielsweise ein pyroelektrisches Detektorarray eingesetzt werden, das mit verschiedenen Interferenzfiltern versehen ist. Auf diese Art und Weise können Absorptionen bei verschiedenen Wellenlängen und somit verschiedener Molekülgruppen detektiert werden. Eine bevorzugte Methode Filter und Lichtquelle auf das Fenster zu bringen ist das Aufdampfen geeigneter Schichten. Bei Verwendung von monochromatischem Licht können Moleküle erfasst werden, die eine Atomkonfiguration besitzen, die genau bei dieser Wellenlänge Licht absorbieren. Durch die Verwendung von mehr als einer Wellenlänge zum Aufbau des evaneszenten Feldes können viele verschiedene Atomkonfigurationen angeregt werden. Es kann durch die Korrelation von verschiedenen Wellenlängenbereichen die Quantifizierung eines Moleküls verbessert werden. Es können auf diese Weise mehrere Molekülgruppen parallel zueinander detektiert werden. Beispiele für verschiedene Analyten, die detektiert werden können sind Glukose, Cholesterin, Creatinin, Harnstoff, Triglyceride.

### System

Der Sensor zur optischen Bestimmung der Konzentration eines Analyten in einer Flüssigkeit, wie er oben ausgeführt wurde, kann auch in einem System verwendet werden. Dieses System beinhaltet weiterhin eine Lichtquelle zur Einkopplung von Strahlung in das Fenster, einen Detektor zum Auffangen der Strahlung an der gegenüberliegenden Seite des Fensters sowie zur Generierung von Messsignalen. Außerdem kann das System ein Auswertegerät zur Berechnung der Konzentration des Analyten aus den Messsignalen beinhalten.

In einer bevorzugten Ausführungsform sind sowohl die Lichtquelle als auch der Detektor auf eine Fläche des Fensters aufgebracht. Sie können dabei entweder verklebt werden oder auf die Fensterfläche aufgedampft sein. Hierbei werden mehrere Schichten von unterschiedlichen Materialien aufgedampft, die zumindest zum Teil elektrisch leitend sind und elektrische Energie in Licht umwandeln können.

In einer alternativen Ausführungsform, in der Lichtquelle und/oder Detektor nicht direkt mit dem Fenster verbunden sind, kann das System weiterhin ein Optomodul beinhalten. Dieses Optomodul dient zur Ankopplung von Lichtleitfasern an das Fenster. Diese Variante ermöglicht den Einsatz von herkömmlichen Detektoren und Lichtquellen, wobei die Handhabung des Systems weniger komfortabel für den Patienten ist. Für den Patienten sind zusätzliche Handhabungsschritte notwendig, wie das Ankoppeln des Optomoduls an den Sensor. Diese Alternative ist dann sehr praktikabel, wenn der Sensor beziehungsweise das System nur selten mit dem Optomodul verbunden wird, was insbesondere bei kurzen Verwendungszyklen der Fall ist. Das System kann also nicht nur für längere Zeiträume zur kontinuierlichen Messung benutzt werden, sondern kann ebenfalls zur Einmalmessung herangezogen werden.

In dem System kann der Sensor zusätzlich mittels eines Haftmediums (z. B. einem Pflaster) auf die Haut des Patienten aufgebracht werden. Der Sensor ist dabei fest mit dem Haftmedium verbunden. Durch etwas Druck auf das Haftmedium werden die mindestens eine Nadelspitze des Nadelarrays in die Haut gedrückt und es kann Flüssigkeit aus der Haut in die Kammer eintreten. Dabei können sowohl Strahler wie auch Detektor so ausgewählt werden, dass sie mechanisch von Steuerelementen abgekoppelt sind. Der Datenaustausch findet dann über elektrische Schnittstellen mit einem separaten Steuer- und Messgerät statt. Eine weitere Möglichkeit ist, die Ankopplung von Lichtleitern an das Fenster, wodurch eine geringere Möglichkeit zur Miniaturisierung ermöglicht wird. Bei dieser Variante ist der Einsatz des Optomoduls vorteilhaft.

In einer bevorzugten Ausführungsform des System kann der Sensor mit einem Optomodul, in Form einer Uhr, integriert sein, wobei die mindestens eine Nadel des Nadelarrays auf der Rückseite der Uhrfläche angebracht ist und beim Tragen der Uhr in die Haut des Patienten eindringen kann. Die Uhr weist dabei eine Auswerteeinheit sowie eine Anzeige für die Ausgewerteten Messdaten auf. Dabei kann das System eine Infrarotschnittstelle zu einem weiteren Messdatenerfassungssystem beinhalten.

### Kurze Beschreibung der Figuren

- Figur 1:: Schematische Darstellung des Nadelarrays in seitlicher Ansicht.
- Figur 2:: Aufsicht auf eine schematische Darstellung des Nadelarrays mit monochromatischer Strahlungsquelle und IR-Detektion.
- Figur 3:: Aufsicht auf das Nadelarray mit polychromatischer Strahlungsquelle und pyroelektrischen Detektorarray.
- Figur 4:: Schematische Darstellung eines Systems bei dem der Sensor inklusive Nadelarray und Fenster mit einem Optikmodul inklusive Messanzeige verbunden ist.
- Figur 5a:: Schematische Darstellung einer Uhr als Messsystem.
- Figur 5b:: Seitliche Ansicht einer Uhr mit integriertem Nadelsensor.
- Figur 5c:: Aufsicht auf die Rückseite einer Uhr mit integriertem Nadelsensor.
- Figur 6a:: Schematische Darstellung eines Pflasters (mit integriertem Nadelarray), das auf die Haut aufgebracht ist und Anschlüsse zum Optomodul aufweist
- Figur 6b:: Seitenansicht eines Opotmoduls in Form einer Uhr mit integrierten Anschlüssen für zum Sensor.
- Figur 6c:: Schematische Darstellung eines Systems bei dem der Sensor inklusive Nadelarray, integriert in ein Pflaster, mit einem Optikmodul in Form einer Uhr verbunden ist.

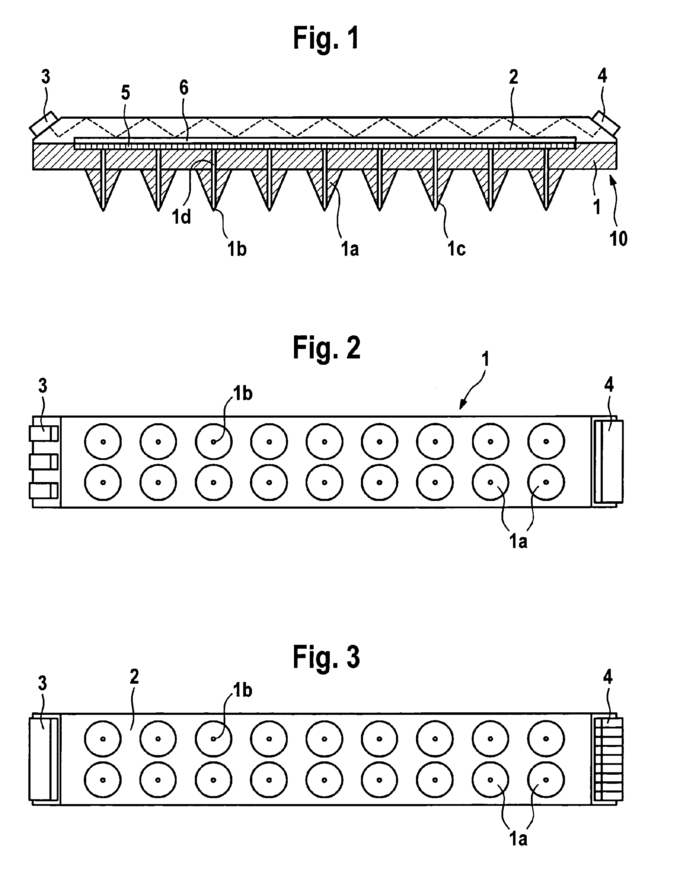

In Figur 1 ist das Prinzip des Nadelarrays in Kombination mit einem Strahlungsfenster dargestellt. Das dargestellte Nadelarray (1), besteht aus einem Silizium Hohlnadelpad (1a). Es ist über eine Membran (5) zur Abtrennung der hochmolekularen Bestandteile (über ca. 10.000 Dalton) fluidisch mit der Messkammer (6) verbunden. Auf der dem Nadelarray (1) gegenüberliegenden Seite der Messkammer (6) befindet sich ein Fenster (2). Dieses Fenster (2) erstreckt sich über die gesamte Länge des Nadelarrays (1). Die mindestens eine Nadel (1a) weist ein distales Ende (1c) und ein proximales Ende (1d) auf. Die Öffnung der Nadel (1b) erstreckt sich dabei vom distalen (1b) zum proximalen Ende (1c) über die gesamte Länge der Nadel (1a). Die Nadeln (1a) können dabei eine eindimensionale Anordnung einnehmen, d. h. in einer Reihe nebeneinander angeordnet sein, dabei können sich in einer Reihe zwischen zwei und 1.000 Nadeln befinden. Eine bevorzugte Ausführungsform ist jedoch die "Nebeneinanderreihung" mehrerer solcher Reihen. Es können z. B. 10 oder mehr solcher Reihen nebeneinander angeordnet sein und eine zweidimensionale Matrix bilden.

Figur 2 zeigt das Nadelarray (1) in Aufsicht. In diesem Beispiel sind 2 Reihen von Nadeln (1a) dargestellt, sie könnten jedoch auch versetzt zueinander angeordnet sein.

An den kurzen Seiten des Fensters (2) ist auf einer Seite eine Lichtquelle (3) aufgebracht. Dies kann beispielsweise ein Infrarot-Strahler wie ein "Quanten Cascaden Laser" (QCL), es können aber auch andere monochromatische Lichtquellen eingesetzt werden. Der Einsatz von nicht monochromatischen Lichtquellen ist alternativ möglich. Dies ist in Figur 3 dargestellt. Hierbei ist jedoch der Einsatz eines Interferenzfilters zwischen Lichtquelle (3) und Fenster (2) nötig. Auf der zweiten kurzen Seite des Fensters ist der Detektor (4) angebracht. Beim Einsatz einer monochromatischen Lichtquelle kann dies beispielsweise ein pyroelektrischer Detektor sein, wie in Figur 2. In Figur 3 ist zur Detektion der verschiedenen Wellenlängen beispielsweise ein pyroelektrisches Detektorarray mit Interferenzfiltern dargestellt.

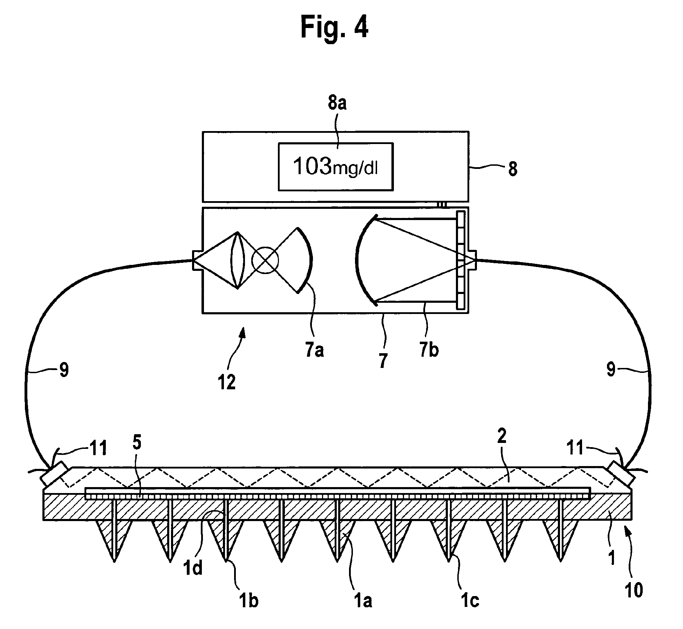

In Figur 4 ist ein System dargestellt, bei dem der Sensor (10) mit dem Optomodul (12) verbunden ist. Das Optomodul (12) besteht aus den Lichtleitern (9), die über ein Klemmelement (11) mit dem Fenster (2) verbunden werden können. Auf der dem Fenster (2) abgewandten Seite, ist ein Lichtleiter (9) mit einer Lichtquelle (7a) und der andere Lichtleiter (9) mit einem Detektor (7b) verbunden. Die Lichtquelle (7a) und der Detektor (7b) befinden sich in einem Gehäuse (7). Die Lichtquelle (7a) und der Detektor (7b) sind mit einem Steuer- und Auswertemodul (8) verbunden, das sowohl die Steuerung der Lichtquelle (7a) als auch des Detektors (7b) übernimmt. Eine weitere Funktion des Auswertemoduls (8) ist die Aufnahme und Auswertung der Lichtsignale vom Detektor (7b). Nach der Auswertung können die Ergebnisse über eine Anzeige (8a) dem Patienten angezeigt werden.

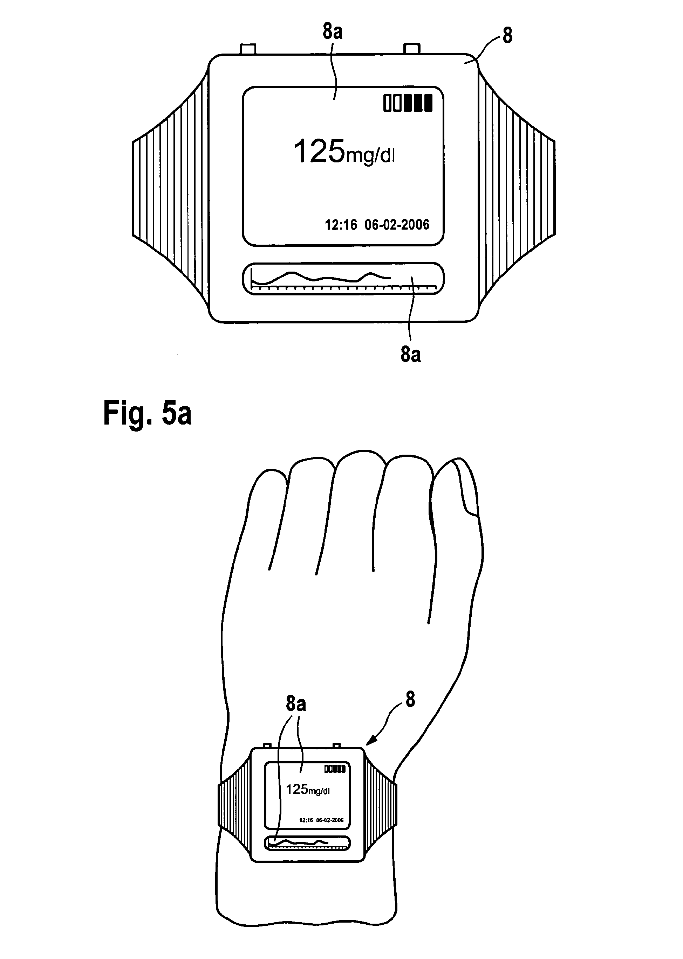

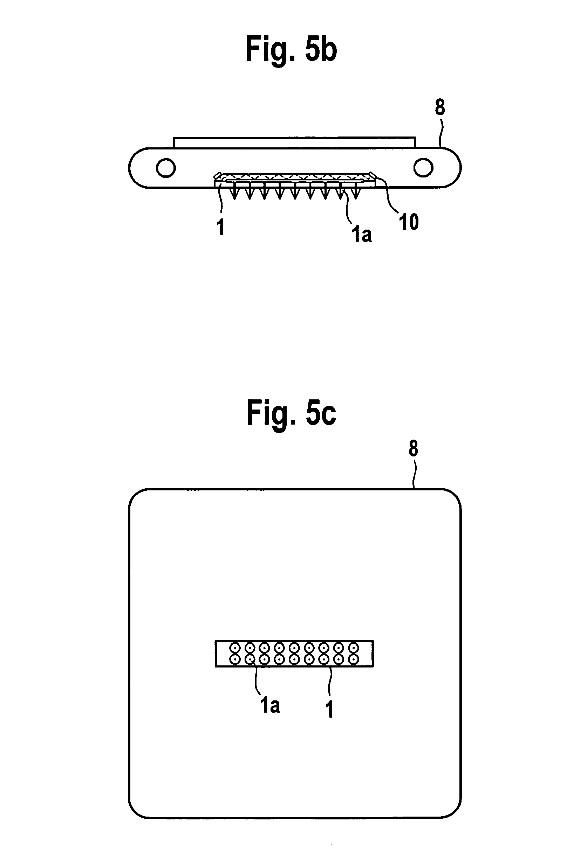

In Figur 5 ist eine bevorzugte Ausführungsform des Sensors (10) dargestellt, bei dem das Nadelarray zusammen mit dem Fenster in ein Optomodul (8) in Form einer Uhr integriert ist. Die Uhr (8) weist ebenfalls eine Anzeige (8a) für die ausgewerteten Messergebnisse auf, wie dies in Figur 5a dargestellt ist. Abbildung 5a ist dabei eine Aufsicht auf die Uhr (8), während Figur 5b eine Seitenansicht der Uhr (8) darstellt. Dabei ist zu erkennen, dass sich auf der Unterseite der Uhr (8) der Sensor (10) einschließlich des Nadelarray (1) befindet. Dabei ragen die Nadeln (1a) aus der Unterseite der Uhr (8) heraus, so dass sie beim Tragen der Uhr (8) in die Haut des Patienten gedrückt werden können. Figur 5c zeigt eine Aufsicht auf die Rückseite der Uhr (8). Das Nadelarray (1) befindet sich dabei auf einem Teil der Rückseitenfläche der Uhr (8).

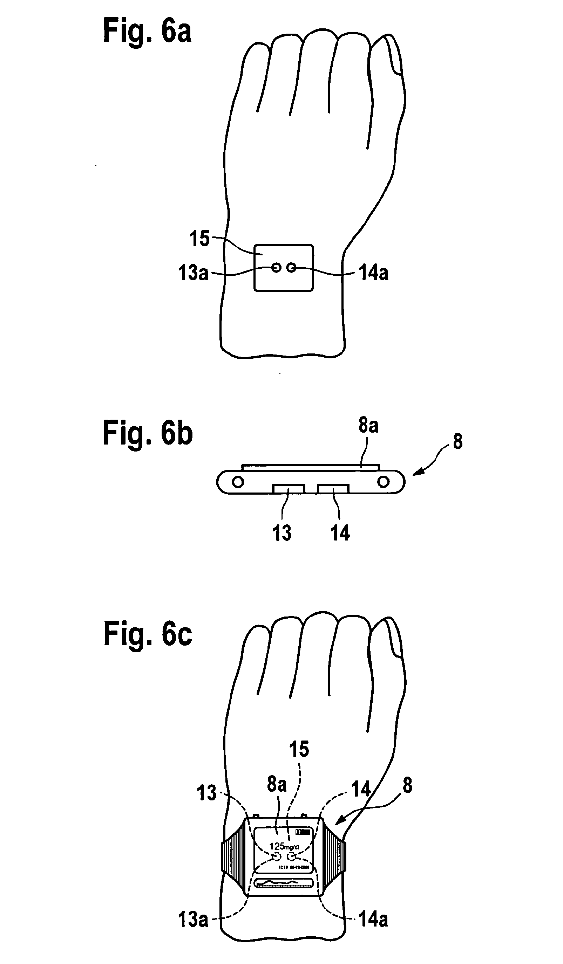

In Figur 6a ist ein Pflaster (15) dargestellt, das auf der Haut eines Patienten angebracht ist. Das Pflaster (15) kann (hier nicht sichtbar) den Sensor (10) enthalten kann. Auf der, der Haut des Patienten gegenüberliegenden Seite sind 2 Anschlüsse (13a) und (14a) angebracht, die entweder optisch oder elektrisch mit einem Optomodul (8) verbunden werden können. Figur 6b zeigt ein Optomodul (8), in Form einer Uhr, in seitlicher Ansicht. Auf der Oberseite ist die Anzeige angeordnet und auf der Unterseite sind zwei Anschlüsse (13) und (14) angeordnet, die dazu dienen das Optomodul (8) mit dem Pflaster (15) und über die Anschlüsse (13) und (14) auch mit dem Sensor (10) zu verbinden. Der Anschluss kann optisch oder elektrisch erfolgen, je nachdem, ob die Lichtquelle und der Detektor integriert im Optomodul oder im Sensor vorliegen. Die Verbindung von Optomodul (8) mit dem Pflaster ist in Figur 6c dargestellt.

## Patentansprüche

1. Sensor zur optischen Bestimmung der Konzentration eines Analyten in einer Körperflüssigkeit, beinhaltend
- eine Nadelanordnung, die mindestens eine Hohlnadel, mit einem sich von dem distalen Ende zum proximalen Ende erstreckenden Hohlraum, aufweist, deren distales Ende zum Stechen geeignet ist und das proximale Ende des Hohlraums in eine Kammer mündet, in der Flüssigkeit gesammelt werden kann, die durch das distale Nadelende eintritt, sowie
- ein Fenster das für Infrarotstrahlung zumindest zum Teil durchlässig ist, und in Kontakt mit der Kammer steht.

2. Sensor gemäß Anspruch 1, **dadurch gekennzeichnet, dass** die Nadelanordnung mehrere Nadeln beinhaltet.

3. Sensor gemäß einem der Ansprüche 1 oder 2, **dadurch gekennzeichnet, dass** das Fenster für Licht verschiedener Wellenlängen durchlässig ist.

4. Sensor gemäß Anspruch 3, **dadurch gekennzeichnet, dass** das der Wellenlängenbereich des Lichts bevorzugt im Bereich von 500 - 20 000 liegt.

5. Sensor gemäß Anspruch 3 oder 4, **dadurch gekennzeichnet, dass** der Wellenlängenbereich des Lichts bevorzugt im Bereich von 2 000 - 12 000 liegt.

6. Sensor gemäß einer der Ansprüche 1 bis 5, **dadurch gekennzeichnet, dass** in der Nadelanordnung ein Filter zur Abtrennung von Molekülen > 10 TDa angeordnet ist.

7. Sensor gemäß einem der Ansprüche 1 bis 6, **dadurch gekennzeichnet, dass** das Volumen der Kammer 1 bis 1000 nl beträgt.

8. Sensor gemäß einem der Ansprüche 1 bis 7, **dadurch gekennzeichnet, dass** der Sensor kein, mit Analyt reagierenden, Reagenz enthält.

9. System, mit einem Sensor gemäß Anspruch 1, weiter beinhaltend eine Lichtquelle zur Einkopplung von Strahlung in das Fenster, einen Detektor zum Auffangen der Strahlung an der gegenüber liegenden Seite des Fensters und Generierung von Messsignalen, sowie ein Auswertegerät zur Berechnung der Konzentration des Analyten aus den Messsignalen.

10. System gemäß Anspruch 9, **dadurch gekennzeichnet, dass** die Lichtquelle Strahlung auf einer Seite des Fensters einstrahlt und der Detektor auf einer anderen Seite des gleichen Fensters die Strahlung aus dem Fenster auffängt.

11. System gemäß Anspruch 9 oder 10, **dadurch gekennzeichnet, dass** ein Optomodul die Lichtquelle und/oder den Detektor mit dem Fenster verbindet.

12. System gemäß Anspruch 9 oder 10, **dadurch gekennzeichnet, dass** ein Membranfilter in die Nadelanordnung eingebaut ist.

13. System gemäß einem der Ansprüche 10 bis 12, **dadurch gekennzeichnet, dass** mindestens ein Teil des Lichtes in dem Fenster total reflektiert wird.
